# EUROPEAN PATENT APPLICATION

(11) **EP 4 600 223 A1**
(43) Date of publication of application: **13.08.2025**
(21) Application number: 24157155.3
(22) Date of filing: 12.02.2024
(51) Int. Cl.: C02F 1/68, E03C 1/046, C02F 1/72, C02F 103/02

(54) **A METHOD FOR DISINFECTING A FLUID SYSTEM**

(71) Applicant: Aspire Technology Group Ltd, St Albans Hertfordshire AL1 3RD (GB)
(72) Inventor: The designation of the inventor has not yet been filed
(74) Representative: Stepney, Gregory John

(57) **Abstract**

A method and system for disinfecting a fluid system is herein described. The method includes providing a fluid system having a reservoir containing a first fluid and a flow path in fluid communication with the reservoir, wherein the fluid system is configured such that the first fluid is selectively releasable from the reservoir to the flow path. A receptacle containing a second fluid, which comprises a disinfectant, is fluidly connected to the fluid system. The fluid system is configured such that the second fluid is substantially retained in the receptacle when the first fluid is not released from the reservoir, and such that the second fluid is drawn into the fluid system from the receptacle when the first fluid is released from the reservoir.

## Description

### FIELD OF THE INVENTION

This invention relates to a disinfection system and a method for disinfecting a fluid system, more particularly wherein the fluid system is a water containing system.

### BACKGROUND

Systems in which water and other fluids flow can become contaminated by a variety of pathogens over time and must accordingly be sterilised. Water systems, and particularly water systems conveying drinking water for human consumption, can be difficult to sterilise adequately due to the challenges associated with accessing the internal surfaces of the pipes of the system. Furthermore, once a system has been cleansed, reinfection can occur requiring further rounds of sterilisation. It can be laborious for users and maintenance workers to have to repeatedly apply disinfectants to these systems. There is therefore a need for a means of disinfecting a fluid system which is convenient to use and which addresses the aforementioned challenges, at least to some extent.

### SUMMARY OF THE INVENTION

In accordance with a first aspect of the invention, there is provided a method for disinfecting a fluid system, the method comprising:
providing a fluid system having a reservoir containing a first fluid and a flow path in fluid communication with the reservoir, wherein the fluid system is configured such that the first fluid is selectively releasable from the reservoir to the flow path;
providing a receptacle containing a second fluid, wherein the second fluid comprises a disinfectant;
fluidly connecting the receptacle to the fluid system; and
releasing the first fluid from the reservoir such that at least a portion of the first fluid flows to the flow path;
wherein the fluid system is configured such that the second fluid is substantially retained in the receptacle when the first fluid is not released from the reservoir, and such that the second fluid is drawn into the fluid system from the receptacle when the first fluid is released from the reservoir.

Advantageously, the method has been found to improve the uniformity of distribution of the disinfectant in the first fluid and in the fluid system, assisting in preventing and/or controlling the growth of harmful microorganisms within the fluid system. In this way, a more consistent disinfection level can be achieved across the entire fluid system. Moreover, the means of disinfecting the fluid system is essentially automated, as the disinfectant is introduced into the fluid system in response to fluid flow, and reduced manual intervention is required, thereby improving efficiency and consistency in treatment.

The selective release of the first fluid from the reservoir promotes a selective release of the second fluid into the fluid system, as the second fluid is only released from the receptacle in response to flow of the first fluid. In this way, the method allows for a substantially "metered" release of the disinfectant, as the disinfectant is periodically released into the fluid system in response to flow of the first fluid. As such, the disinfectant can last longer before the receptacle becomes depleted, and the disinfectant can be repeatedly introduced into the fluid system in response to flow, improving the distribution of the disinfectant.

It will be understood that the first fluid will have a static pressure in the reservoir which prevents significant flow of the second fluid from the receptacle when the first fluid is stationary. In contrast, when the first fluid flows, the pressure differential and dynamic pressure in the system promotes the flow of the second fluid from the receptacle into the fluid system. In this way, the second fluid is selectively released into the fluid system, i.e. in response to flow of the first fluid.

The first fluid may be released from the reservoir for a predetermined time, and the first fluid may be prevented from release from the reservoir after the predetermined time.

The first fluid may comprise water, oil, coolant, emulsified coolant, lubricant, cutting fluid, heat transfer fluid, mineral oil such as petroleum-based oil, neatsfoot oil, silicone oil, vegetable oil, fluorocarbon oil, polyalkylene glycol, hydrocarbon-based liquid such as kerosene, paraffin, ethanol, isopropanol, fracking fluid or combinations thereof.

The second fluid may comprise an aqueous composition comprising one or more peroxides. The one or more peroxides may comprise at least one of hydrogen peroxide, peracetic acid, performic acid, peroxymonosulfuric acid, peroxynitric acid, and peroxymonophosphoric acid. For example, the one or more peroxides may comprise at least one of hydrogen peroxide and peracetic acid, or a mixture of hydrogen peroxide and peracetic acid.

The peroxide may be present in the composition in an amount of from 5 to 50 wt%, preferably 10 to 40 wt%. This range represents the most effective and safe peroxide concentration range for a ready-to-use disinfectant.

The second fluid in the receptacle may be pressurised to a pressure of from 100 to 1500 kPa (absolute), preferably 400 to 1200 kPa (abolute) or 500 to 800 kPa (absolute). Preferably the internal pressure of the receptacle is higher than the pressure in the flow path. Preferably the pressure of the first fluid is atmospheric pressure (about 100 kPa (absolute)). Advantageously, pressurising the second fluid has been found to create turbulence in the fluid system when the second fluid is drawn into the fluid system in response to flow of the first fluid. The presence of turbulent flow in the fluid system improves mixing between the second fluid and the first fluid, further improving the distribution of the disinfectant in the fluid system.

An internal surface of the receptacle may be coated with a polyamide-imide (PAM) coating. Where the second fluid comprises an aqueous composition comprising one or more peroxides, the PAM coating may provide a stabilising effect on the peroxides which permits the second fluid to be stored for extended periods of time without appreciable degradation of the one or more peroxides. The PAM coating provides a barrier between the receptacle wall and second fluid which, in embodiments in which the receptacle comprises a metal, avoids metal-catalysed decomposition of the peroxide.

The receptacle may comprise aluminium or an aluminium alloy. Aluminium is a preferred material for the receptacle due to its low weight, structural rigidity and recyclability. Although aluminium can react with peroxide, the PAM coating provides a barrier which prevents contact between the aluminium and peroxide.

The receptacle may be impervious to light. By preventing light from entering the interior of the receptacle, UV-induced degradation of the peroxide can be reduced and the shelf life of the second fluid extended.

The receptacle may contain a propellant. The propellant may be selected from one or more of nitrogen, dimethyl ether (DME), propane, n-butane, and iso-butane. The propellant may contribute to generating turbulent mixing of the second fluid with the first fluid.

The fluid system may comprise a valve and the receptacle may be fluidly connected to the fluid system via the valve. Advantageously, the valve can assist in controlling flow of the second fluid into the first fluid, while also assisting in isolating the two fluids prior to mixing.

The valve may be a needle valve. Advantageously, a needle valve can provide for precise flow control, with the valve disc allowing for gradual adjustments, improving the ease at which flow of the second fluid into the first fluid can be regulated.

The valve may be a non-return valve configured to prevent flow into the receptacle. Advantageously, the second fluid in the receptacle is isolated until the fluid is released therefrom, preventing any harmful microorganisms from entering the receptacle, and promoting the mixing of the second fluid and first fluid that occurs in the fluid system.

The valve may be configured to move between a closed configuration, in which the second fluid is retained in the receptacle, and an open configuration, in which the second fluid flows from the receptacle to the fluid system, and the valve may be configured to move between the closed configuration and the open configuration in response to a pressure change in the fluid system.

The valve may be positioned proximate the flow path such that the second fluid is drawn into the flow path. Advantageously, positioning the valve proximate the flow path can facilitate the introduction of the fluid directly into the first fluid flowing in the flow path, increasing mixing of disinfectant with the first fluid within the fluid system.

The step of fluidly connecting the receptacle to the fluid system comprises removeably mounting the receptacle to the fluid system. Advantageously, the receptacle can be simply connected to and/or removed from the fluid system, facilitating retrofitting of an existing fluid system with the receptacle, whilst also facilitating simple removal/replacement of the receptacle once the second fluid has been depleted.

The fluid system may comprise at least one flow actuator configured to release the first fluid from the reservoir, the flow actuator comprising a pump system and/or a valve system. The flow actuator may be user actuated.

The fluid system may comprise an open or closed water system, a plumbing system, an irrigation system, a water sanitation system, a cooling circuit, a Heating, Ventilation, and Air Conditioning (HVAC) system, an engine cooling system, a drinking water system, a reverse osmosis unit, a humidifier, or a nano or ultra filtration water system.

In accordance with a second aspect of this invention, there is provided a disinfection system comprising:
a fluid system having a reservoir for a first fluid, and a flow path in fluid communication with the reservoir, wherein the fluid system is configured such that the first fluid is selectively releasable from the reservoir to the flow path;
a receptacle configured to be fluidly connected to the fluid system, wherein the receptacle contains a second fluid comprising a disinfectant;
wherein the fluid system is configured to substantially retain the second fluid in the receptacle when the first fluid is not released from the reservoir, and to permit the second fluid to be drawn into the fluid system from the receptacle when the first fluid is released from the reservoir.

The features and advantages associated with the first aspect apply equally to the second aspect.

Unless otherwise stated, each of the integers described may be used in combination with any other integer as would be understood by the person skilled in the art. Further, although all aspects of the invention preferably "comprise" the features described in relation to that aspect, it is specifically envisaged that they may "consist" or "consist essentially" of those features outlined in the claims. In addition, all terms, unless specifically defined herein, are intended to be given their commonly understood meaning in the art.

As used herein and in the accompanying claims, unless the context requires otherwise, "comprise" or variations such as "comprises" or "comprising" will be understood to imply the inclusion of a stated integer or group of integers but not the exclusion of any other integer or group of integers.

Further, in the discussion of the invention, unless stated to the contrary, the disclosure of alternative values for the upper or lower limit of the permitted range of a parameter is to be construed as an implied statement that each intermediate value of the said parameter, lying between the smaller and greater of the alternatives, is itself also disclosed as a possible value for the parameter.

In addition, unless otherwise stated, all numerical values appearing in this application are to be understood as being modified by the term "about". As used herein, the term "about" means that the stated value can vary by ± 10%. For example, about 90 wt% means 90±9 wt%, and about 0.1 wt% means 0.1±0.01 wt%. When used with reference to a range, the term "about" applies to all values in the range.

In order that the invention may be more readily understood, it will be described further with reference to the figures and to the specific examples hereinafter.

### DETAILED DESCRIPTION OF THE INVENTION

The present disclosure provides a method for disinfecting a fluid system, such as a system in which water flows and which may become contaminated by biological pathogens. Examples of such systems include open or closed water systems, plumbing systems, irrigation systems, water sanitation systems, cooling circuits (e.g. for a vehicle engine), Heating, Ventilation, and Air Conditioning (HVAC) systems, drinking water dispensers, reverse osmosis systems, humidifiers, and nano or ultra filtration water systems.

The method includes providing a fluid system having a reservoir containing a first fluid and a flow path in fluid communication with the reservoir, wherein the fluid system is configured such that the first fluid is selectively releasable from the reservoir to the flow path. A receptacle containing a second fluid, which comprises a disinfectant, is fluidly connected to the fluid system. The fluid system is configured such that the second fluid is substantially retained in the receptacle when the first fluid is not released from the reservoir, and such that the second fluid is drawn into the fluid system from the receptacle when the first fluid is released from the reservoir.

The first fluid may be released from the reservoir for a predetermined time, and the first fluid may be prevented from release from the reservoir after the predetermined time. The predetermined time will depend on the particular system and may range from a few seconds (e.g. 1, 2, 3, 4, 5, 6, 7, 8, 9, 10, 20, or 30 seconds) to several minutes (e.g. 1, 2, 3, 4, 5, 6, 7, 8, 9 or 10 minutes). The first fluid preferably comprises water, since water-based fluids are more easily contaminated by microorganisms. However, in some embodiments, the fluid may comprise organic solvents such as methanol ethanol or isopropanol, refrigerants, coolants, emulsified coolants, lubricants, mineral oils, silicone oils, vegetable oils, neatsfoot oil, fluorocarbon oils, heat transfer fluids, cutting fluids, glycols, fracking fluids and the like.

The second fluid may comprise an aqueous composition comprising one or more peroxides. The one or more peroxides may comprise at least one of hydrogen peroxide, peracetic acid, performic acid, peroxymonosulfuric acid, peroxynitric acid, and peroxymonophosphoric acid. For example, the one or more peroxides may comprise at least one of hydrogen peroxide and peracetic acid, or a mixture of hydrogen peroxide and peracetic acid.

The peroxide may be present in the composition in an amount of from 5 to 50 wt%. In some embodiments, the peroxide may be present in the composition in an amount of from 10 to 40 wt%, from 15 to 35 wt%, from 20 to 30 wt%, or about 25 wt%.

The second fluid may comprise one or more solvents such as water, acetonitrile, methanol, ethanol, propanol, isopropanol, butanol or isobutanol, and an adjuvant such as an organic acid (e.g. formic acid, acetic acid, or propionic acid).

The second fluid in the receptacle may be pressurised to a pressure of from 100 to 1500 kPa (absolute), preferably 400 to 1200 kPa (absolute) or 500 to 800 kPa (absolute). Preferably the internal pressure of the receptacle is higher than the pressure in the flow path. For example, the internal pressure of the receptacle may be from about 1200 kPa (absolute) to about 400 kPa (absolute), from 100 kPa (absolute)to about 400 k Pa (absolute), or from about 500 kPa (absolute) to about 800 kPa (absolute). The receptacle may contain a propellant for ejecting the second fluid. The propellant may be selected from one or more of nitrogen, dimethyl ether (DME), propane, n-butane, and iso-butane. In some embodiments, the propellant may be dimethyl ether (DME). The propellant may be present in the receptacle in an amount of from 1 wt% to 99 wt%, from 5 wt% to 80 wt%, from 10 wt% to 60 wt%, from 15 wt% to 50 wt%, from 20 wt% to 40 wt%, from 20 wt% to 30 wt%, or about 25 wt%, based on the combined weight of components in the receptacle. Preferably the propellant is present in an amount of from 20 wt% to 40 wt%, or about 25 wt%.

An internal surface of the receptacle may be coated with a polyamide-imide (PAM) coating. The PAM coating provides a barrier between the receptacle wall and second fluid which, in embodiments in which the receptacle comprises a metal and the second fluid comprises one or more peroxides, avoids metal-catalysed decomposition of the peroxide. Advantageously, the PAM coating may provide a stabilising effect which permits the second fluid to be stored for extended periods of time without appreciable degradation of the one or more peroxides. The PAM may coat the entire internal surface of the receptacle. In some embodiments, the PAM coating may comprise gold pam-2-c-internal liner 8460 N. An example of a PAM coating which is suitable for use in the present invention is described in WO2014096075A1, the contents of which is incorporated herein by reference.

The receptacle may comprise aluminium or an aluminium alloy. Aluminium is a preferred material for the receptacle due to its low weight, structural rigidity and recyclability.

The fluid system may comprise a valve for controlling flow of the second fluid into the first fluid and the receptacle may be fluidly connected to the fluid system via the valve. The valve may also assist in isolating the two fluids prior to mixing. The valve may be activated (i.e. opened) when a reduction in pressure in the fluid system resulting from discharge of the first fluid reaches a threshold pressure. The threshold may be a drop in pressure of greater than 10, 20, 30, 40, 50, 100, 200, 300, 400, 500, 1000, 2000, 3000, 4000, or 5000 kPa (absolute), or between any combination of two of these pressures. Alternatively, the valve may be activated when a pressure differential between the interior of the receptacle and the flow path exceeds 10, 20, 30, 40, 50, 100, 200, 300, 400, 500, 1000, 2000, 3000, 4000, or 5000 kPa (absolute), or where the pressure differential is between any two of these pressures.

The valve may be a needle valve, which may provide for precise flow control and improve the ease at which flow of the second fluid into the first fluid can be regulated. The valve may be a non-return valve configured to prevent flow into the receptacle so that the second fluid is isolated from the first fluid until the first fluid is released. The valve may be configured to move between a closed configuration, in which the second fluid is retained in the receptacle, and an open configuration, in which the second fluid flows from the receptacle to the fluid system, and the valve may be configured to move between the closed configuration and the open configuration in response to a pressure change in the fluid system. The valve may be positioned proximate the flow path such that the second fluid is drawn into the flow path to facilitate the introduction of the second fluid directly into the first fluid flowing in the flow path.

The step of fluidly connecting the receptacle to the fluid system may comprise removably mounting the receptacle to the fluid system. The receptacle can be simply connected to and/or removed from the fluid system, facilitating retrofitting of an existing fluid system with the receptacle, whilst also facilitating simple removal/replacement of the receptacle once the second fluid has been depleted. The valve may comprise engagement formations configured to engage complementary formations on the receptacle, so that the receptacle can be releasably secured against the valve and held securely in position. The engagement formations may comprise screw thread, a twist-lock arrangement, a snap-fit connector, or any other suitable connector capable of holding the receptacle in place.

The fluid system may comprise at least one flow actuator configured to release the first fluid from the reservoir, the flow actuator comprising a pump system and/or a valve system. The flow actuator may be user actuated. The nature of the flow actuator will depend on the particular system used. For example, where the system is a plumbing system, the flow actuator may comprise a toilet flush or a water tap.

The fluid system may comprise an open or closed water system, a plumbing system, an irrigation system, a water sanitation system, a cooling circuit, a Heating, Ventilation, and Air Conditioning (HVAC) system, an engine cooling system, a drinking water system, a reverse osmosis unit, a humidifier, or a nano or ultra filtration water system.

The present invention further provides a disinfection system comprising a fluid system having a reservoir for a first fluid, and a flow path in fluid communication with the reservoir. The fluid system is configured such that the first fluid is selectively releasable from the reservoir to the flow path. A receptacle containing a second fluid comprising a disinfectant is configured to be fluidly connected to the fluid system. The fluid system is configured to substantially retain the second fluid in the receptacle when the first fluid is not released from the reservoir, and to permit the second fluid to be drawn into the fluid system from the receptacle when the first fluid is released from the reservoir.

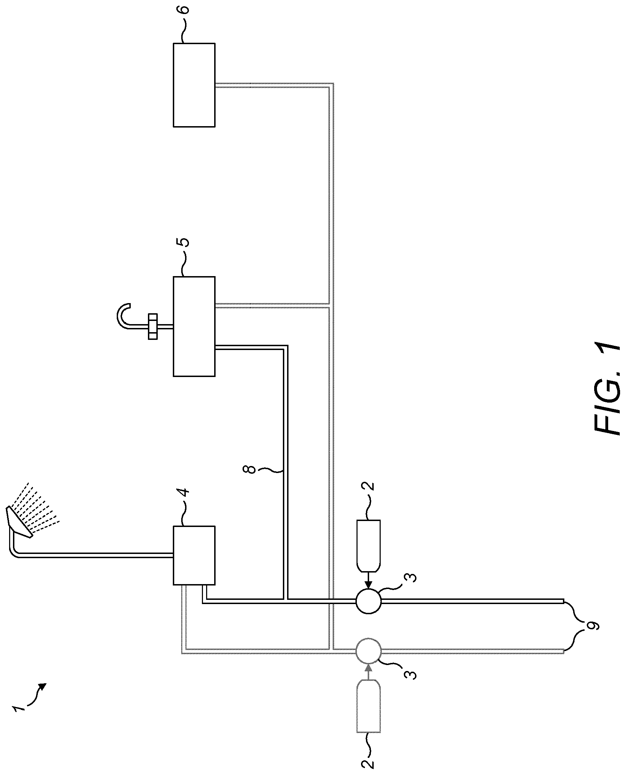

In one embodiment as shown in Figure 1, the system (1) may be a plumbing system including a reservoir such as a main water supply or water tank (9), a shower (4), a sink (5), a toilet comprising a cistern (6), a first fluid comprising water, and a flow path defined by pipes (8) connecting the reservoir (9) to the shower (4), the sink (5), and the toilet comprising a cistern (6). The system (1) may also include a receptacle (2) comprising a second fluid comprising a disinfectant releasably connected to the pipes (8) via a valve (3). In use, flushing of the toilet releases the first fluid comprising water from the cistern (6) into the pipes (8). The flow of the first fluid generates a pressure differential and dynamic pressure in the system (1) which activates the flow of the second fluid from the receptacle (2) into the pipes (8). In this way, the second fluid is selectively released into the system (1) in response to flow of the first fluid from the cistern (6), resulting in disinfection of the first fluid and the pipes (8) by the action of the second fluid comprising a disinfectant.

In another embodiment the system may be an irrigation system comprising a flow path defined by irrigation pipes, a first fluid comprising water, a receptacle and a second fluid comprising a disinfectant. When the first fluid is turned on and flows through the irrigation pipes, the receptacle, which is connected to the irrigation pipes, is activated to release the second fluid comprising a disinfectant which sterilises the first fluid comprising water and the irrigation pipes.

The invention will now be described in further detail regarding the following non-limiting examples.

### EXAMPLES

### Example 1:

The disinfection methods of the present invention were tested in a water system in accordance with Figure 1.

The water system was tested for bacteria prior to release of any disinfectant. The water system was tested again after disinfectant had been released from the receptacle and it was found that the following bacteria has been effectively removed from the system:
- *Cupriavidus pauculus*
- *Legionella* spp.
- *Pseudomonas fluorescens*
- *Pseudomonas aeruginosa*
- *Stenotrophomonas maltophilia*
- *Acinetobacter ursingii*
- *Enterobacter cloacae*
- *Klebsiella oxytoca*
- *Serratia marcescens*
- *Pseudomonas putida*
- *Pantoea* spp.
- *Klebsiella pneumonia*
- *Chryseomonas indologenes*

The removal of the above bacteria was independently verified.

The results show that the disinfection methods of the present invention are effective against a wide range of bacteria.

It would be appreciated that the method and system of the invention are capable of being implemented in a variety of ways, only a few of which have been illustrated and described above.

## Claims

1. A method for disinfecting a fluid system, the method comprising:
providing a fluid system having a reservoir containing a first fluid and a flow path in fluid communication with the reservoir, wherein the fluid system is configured such that the first fluid is selectively releasable from the reservoir to the flow path;
providing a receptacle containing a second fluid, wherein the second fluid comprises a disinfectant;
fluidly connecting the receptacle to the fluid system; and
releasing the first fluid from the reservoir such that at least a portion of the first fluid flows to the flow path;
wherein the fluid system is configured such that the second fluid is substantially retained in the receptacle when the first fluid is not released from the reservoir, and such that the second fluid is drawn into the fluid system from the receptacle when the first fluid is released from the reservoir.

2. The method according to claim 1, wherein the first fluid is released from the reservoir for a predetermined time, and wherein the first fluid is prevented from release from the reservoir after the predetermined time.

3. The method according to claim 1 or 2, wherein the disinfectant comprises a peroxide, preferably wherein the peroxide is selected from hydrogen peroxide and peracetic acid.

4. The method according to any preceding claim, wherein the second fluid in the receptacle is pressurised to a pressure of from 400 to 1200 kPa (absolute).

5. The method according to any preceding claim, wherein the fluid system comprises a valve and wherein the receptacle is fluidly connected to the fluid system via the valve.

6. The method according to claim 5, wherein the valve is a needle valve.

7. The method according to claim 5 or 6, wherein the valve is a non-return valve configured to prevent flow into the receptacle.

8. The method according to any one of claims 5 to 7, wherein the valve is configured to move between a closed configuration, in which the second fluid is retained in the receptacle, and an open configuration, in which the second fluid flows from the receptacle to the fluid system, and wherein the valve is configured to move between the closed configuration and the open configuration in response to a pressure change in the fluid system.

9. The method according to any one of claims 5 to 9, wherein the valve is positioned proximate the flow path such that the second fluid is drawn into the flow path.

10. The method according to any preceding claim, wherein the step of fluidly connecting the receptacle to the fluid system comprises removeably mounting the receptacle to the fluid system.

11. The method according to any preceding claim, wherein the fluid system comprises at least one flow actuator configured to release the first fluid from the reservoir, the flow actuator comprising a pump system and/or a valve system.

12. The method according to claim 11, wherein the flow actuator is user actuated.

13. The method according to claim 12, wherein the first fluid comprises water, oil, coolant, emulsified coolant, lubricant, cutting fluid, heat transfer fluid, mineral oil such as petroleum-based oil, neatsfoot oil, silicone oil, vegetable oil, fluorocarbon oil, polyalkylene glycol, hydrocarbon-based liquid such as kerosene, paraffin, ethanol, isopropanol, fracking fluid or combinations thereof.

14. A disinfection system comprising:
a fluid system having a reservoir for a first fluid, and a flow path in fluid communication with the reservoir, wherein the fluid system is configured such that the first fluid is selectively releasable from the reservoir to the flow path;
a receptacle configured to be fluidly connected to the fluid system, wherein the receptacle contains a second fluid comprising a disinfectant;
wherein the fluid system is configured to substantially retain the second fluid in the receptacle when the first fluid is not released from the reservoir, and to permit the second fluid to be drawn into the fluid system from the receptacle when the first fluid is released from the reservoir.
